# EUROPÄISCHE PATENTSCHRIFT

(11) **EP 2 952 256 B1**
(45) Veröffentlichungstag und Bekanntmachung des Hinweises auf die Patenterteilung: **17.05.2017**
(21) Anmeldenummer: 15170317.0
(22) Anmeldetag: 02.06.2015
(51) Int. Cl.: B01J 31/10, C08F 8/36, C08K 5/46, C08F 212/08, B01J 47/00, C07C 37/20, C07C 39/16

(54) **VERFAHREN ZUR HERSTELLUNG VON KATALYSATOREN**
METHOD FOR THE PRODUCTION OF CATALYSTS
PROCÉDÉ DE FABRICATION DE CATALYSEURS

(30) Priorität: 05.06.2014 EP 14171224
(43) Veröffentlichungstag der Anmeldung: 09.12.2015
(73) Patentinhaber: LANXESS Deutschland GmbH, 50569 Köln (DE)
(72) Erfinder: Klipper, Reinhold, 50933 Köln (DE); Vanhoorne, Pierre, 40789 Monheim (DE); Wagner, Rudolf, 51061 Köln (DE); Mittag, Hubertus, 06766 Bitterfeld-Wolfen (DE)

(56) Entgegenhaltungen:
- EP-A1- 2 497 574
- DE-A1- 2 164 339
- US-A- 5 698 600
- US-A1- 2012 283 485

## Beschreibung

Die vorliegende Erfindung betrifft Katalysatoren für Kondensations-, Additions- und Veresterungsreaktionen ein Verfahren zur Herstellung dieser Katalysatoren und deren Verwendung zur Herstellung von Bisphenolen.

Die Kondensation von Phenolen und Ketonen zu Bisphenolen spielt in industriellen Herstellungsverfahren eine hervorzuhebende Rolle. Insbesondere Bisphenol A dient u.a. zur Herstellung von Polycarbonat und wird durch Kondensation von Phenol und Aceton in Gegenwart von Chlorwasserstoff oder Polystyrolsulfonsäuren als Katalysatoren hergestellt. Die eingesetzten Polystyrolsulfonsäuren sind stark saure Kationenaustauscher, die neutralisiert werden müssen. Häufig geschieht dies durch Zugabe eines sogenannten Promoters, z.B. eines Mercaptans, in Reaktoren unter starkem Rühren. Allerdings sind technische Reaktoren mit entsprechend großen und umfassenden Rührvorrichtungen selten und die Durchmischung bzw. gleichmäßige Beschichtung der Katalysatoren mit den Promotoren ist noch unzureichend.

Eine Möglichkeit den o.g. Nachteil zu überwinden besteht darin, die Dotierung im Rahmen des Herstellungsverfahrens der stark sauren Kationenaustauscher vorzunehmen.

Aus der EP 0486277 A ist z.B. ein Verfahren zur Herstellung eines dotierten Bisphenol A Katalysators bekannt, bei dem ein Styrol-Divinylbenzol basierter stark saurer Kationenaustauscher in Gegenwart von Schwefelsäure und einem halogenhaltigen Quellungsmittel sulfoniert wird und danach mit einem Mercaptanpromoter dotiert wird.

Weitere Verfahren zur Herstellung von Bisphenol A Katalysatoren auf u.a. Basis von sulfonierten Styrol-Divinylbenzol Copolymerisaten mittels Dotierung durch Promotoren sind aus der WO2008/157025 A oder der DE 2164339 B bekannt.

Allen bekannten Verfahren ist gemeinsam, dass die Katalysatoren weiterhin eine mangelnde Reinheit besitzen und/oder die Katalysatoraktivität nicht ausreichend ist.

Es bestand daher ein Bedürfnis nach einem Verfahren zur Herstellung eines Katalysators, mit dem die Nachteile des Standes der Technik überwunden werden können. Überraschend wurde nun gefunden, dass mit Hilfe des erfindungsgemäßen Verfahrens Katalysatoren hergestellt werden können, die eine höhere Totalkapazität aufweisen und weniger organische Verunreinigungen enthalten, als Katalysatoren, die nach herkömmlichen Herstellungsverfahren in Gegenwart eines Quellungsmittels hergestellt werden.

Gegenstand der Erfindung ist daher ein Verfahren zur Herstellung eines Katalysators, bei dem,
a) Monomertröpfchen aus einem Gemisch enthaltend mindestens eine monoethylenisch ungesättigte, aromatische Verbindung, mindestens eine multiethylenisch ungesättigte Verbindung, mindestens einen Initiator zu einem vernetzten Perlpolymerisat umgesetzt werden
   und
b) das vernetzte Perlpolymerisat aus Schritt a) bei einer Temperatur von 50 °C bis 160 °C in Gegenwart von Schwefelsäure sulfoniert wird und die Konzentration der Schwefelsäure während der Reaktion mindestens 75 Gew. % beträgt und die Menge der eingesetzten Schwefelsäure 70 Gew. % bis 95 Gew.% und die Menge des eingesetzten Perlpolymerisates 5 Gew. % bis 30 Gew. % bezogen auf die Gesamtmenge an eingesetzter Schwefelsäure und Perlpolymerisat beträgt und die Schwefelsäure in einer Konzentration zwischen 92 % und 99 % eingesetzt wird,und die Summe der Gewichtsprozente aus Schwefelsäure und Perlpolymerisat bezogen auf die Menge der Reaktionsmischung > 98 Gew.-% ist und
c) die sulfonierten, vernetzten Perlpolymerisate aus Schritt b) mit mindestens einer schwefelhaltigen Verbindung aus der Gruppe Thioalkohole, Thioether und Thioester oder Gemische dieser Verbindungen umgesetzt werden.

Erfindungsgemäß geeignete vernetzte Perlpolymerisate sind Copolymerisate aus mindestens einer monoethylenisch ungesättigten aromatischen Verbindung und mindestens einer multiethylenisch ungesättigte Verbindung.

Als monoethylenisch ungesättigte aromatische (= vinylaromatische) Verbindungen in Schritt a) werden bevorzugt Styrol, α-Methylstyrol, Vinyltoluol, Ethylstyrol, t-Butylstyrol, Chlorstyrol, Bromstyrol, Chlormethylstyrol oder Vinylnaphthalin eingesetzt. Gut geeignet sind auch Mischungen dieser Monomere. Besonders bevorzugt sind Styrol und Vinyltoluol.

Die multiethylenisch ungesättigte Verbindungen in Schritt a) dienen als Vernetzer. Als multiethylenisch ungesättigte Verbindungen in Schritt a) werden bevorzugt Divinylbenzol, Divinyltoluol, Trivinylbenzol, Octadien oder Triallylcyanurat eingesetzt. Besonders bevorzugt sind die multiethylenisch ungesättigten Verbindungen, vinylaromatische Verbindungen, wie insbesondere Divinylbenzol und Trivinylbenzol. Ganz besonders bevorzugt ist Divinylbenzol. Zur Herstellung der Perlpolymerisate können technische Qualitäten von Divinylbenzol eingesetzt werden, die neben den Isomeren des Divinylbenzols übliche Nebenprodukte wie Ethylvinylbenzol enthalten. Erfindungsgemäß sind technische Qualitäten mit Divinylbenzolgehalten von 55 bis 85 Gew. % besonders gut geeignet. Die multiethylenisch ungesättigten Verbindungen können alleine oder als Gemisch verschiedener multiethylenisch ungesättigter Verbindungen eingesetzt werden.

Die Gesamtmenge einzusetzender multiethylenisch ungesättigter Verbindungen in Schritt a) beträgt in der Regel 0,5 bis 6 Gew.-% bezogen auf die Summe der ethylenisch ungesättigten Verbindungen. Es können aber ebenfalls kleinere oder größere Mengen eingesetzt werden. Die Gesamtmenge einzusetzender multiethylenisch ungesättigter Verbindungen in Schritt a) beträgt bevorzugt 1,5 bis 5 Gew.-%, besonders bevorzugt 1 bis 4 Gew.-%, bezogen auf die Summe der ethylenisch ungesättigten Verbindungen.

Bevorzugt wird in Schritt a) ein Gemisch aus Styrol und Divinylbenzol eingesetzt.

Zur Herstellung der vernetzten Perlpolymerisate im Schritt a) werden die oben genannten ethylenisch ungesättigten Verbindungen (Monomere) in einer weiteren bevorzugten Ausführungsform der vorliegenden Erfindung in Anwesenheit eines Dispergierhilfsmittels unter Verwendung eines Initiators in wässriger Suspension polymerisiert.

Als Dispergierhilfsmittel werden bevorzugt natürliche und synthetische wasserlösliche Polymere eingesetzt. Besonders bevorzugt werden Gelatine, Cellulosederivate, Stärke, Polyvinylalkohol, Polyvinylpyrrolidon, Polyacrylsäure, Polymethacrylsäure oder Copolymerisate aus (Meth)acrylsäure und (Meth)acrylsäureestern eingesetzt. Ganz besonders bevorzugt werden Gelatine und Cellulosederivate, insbesondere Celluloseester und Celluloseether, wie Carboxymethylcellulose, Methylcellulose, Hydroxyethylcellulose oder Methylhydroxy-ethylcellulose eingesetzt. Die Einsatzmenge der Dispergierhilfsmittel beträgt im Allgemeinen 0,05 bis 1 %, vorzugsweise 0,1 bis 0,5 %, bezogen auf die Wasserphase.

Im Schritt a) in der vorliegenden Erfindung werden im Monomerengemisch Initiatoren eingesetzt. Als Monomergemisch wird in der vorliegenden Erfindung das Gemisch aus monoethylenisch ungesättigte(n), aromatische(n) Verbindung(en) und multiethylenisch ungesättigte(n) Verbindung(en), bezeichnet. Geeignete Initiatoren sind Verbindungen, die bei Temperaturerhöhung freie Radikale bilden und sich im Monomergemisch lösen. Bevorzugt werden Peroxyverbindungen, besonders bevorzugt Dibenzoylperoxid, Dilaurylperoxid, Bis-(p-chlorbenzoyl)peroxid, Dicyclohexylperoxydicarbonat oder tert.-Amylperoxy-2-ethylhexan sowie Azoverbindungen, besonders bevorzugt 2,2'-Azobis(isobutyronitril) oder 2,2'-Azobis(2-methylisobutyronitril) oder auch aliphatische Peroxyester, bevorzugt tert.-Butylperoxyacetat, tert.-Butylperoxyisobutyrat, tert.-Butylperoxypivalat, tert.-Butylperoxyoctoat, tert.-Butylperoxy-2-ethylhexanoat, tert.-Butylperoxyneodecanoat, tert.-Amylperoxypivalat, tert.-Amylperoxyoctoat, tert.-Amyl-peroxy-2-ethylhexanoat, tert.-Amylperoxyneodecanoat, 2,5-Bis(2-ethylhexanoylperoxy)-2,5-dimethylhexan, 2,5-Dipivaloyl-2,5-dimethylhexan, 2,5-Bis(2-neodecanoylperoxy)-2,5-dimethylhexan, Di-tert.-butylperoxyazelat oder Di-tert.-amylperoxyazelat eingesetzt.

Die im Monomergemisch löslichen Initiatoren werden im Allgemeinen in Mengen von 0,05 bis 6,0 Gew.-% bezogen auf die Summe der ethylenisch ungesättigten Verbindungen eingesetzt. Es können aber ebenfalls kleinere oder größere Mengen eingesetzt werden. Die im Monomergemisch löslichen Initiatoren werden vorzugsweise in Mengen von 0,1 bis 5,0 Gew.-%, besonders bevorzugt 0,2 bis 2 Gew.-%, bezogen auf die Summe der ethylenisch ungesättigten Verbindungen, angewendet.

Die Wasserphase kann ein Puffersystem enthalten, welches den pH-Wert der Wasserphase auf einen Wert zwischen 12 und 3, vorzugsweise zwischen 10 und 4 einstellt. Besonders gut geeignete Puffersysteme enthalten Phosphat-; Acetat-, Citrat- oder Boratsalze.

Es kann vorteilhaft sein, einen in der wässrigen Phase gelösten Inhibitor einzusetzen. Als Inhibitoren kommen sowohl anorganische als auch organische Stoffe in Frage. Beispiele für anorganische Inhibitoren sind Stickstoffverbindungen wie Hydroxylamin, Hydrazin, Natriumnitrit oder Kaliumnitrit. Beispiele für organische Inhibitoren sind phenolische Verbindungen wie Hydrochinon, Hydrochinonmonomethylether, Resorcin, Brenzkatechin, tert.-Butylbrenzkatechin, Kondensationsprodukte aus Phenolen mit Aldehyden. Weitere organische Inhibitoren sind stickstoffhaltige Verbindungen wie z.B. Diethylhydroxylamin und Isopropylhydroxylamin. Resorcin wird als Inhibitor bevorzugt. Die Konzentration des Inhibitors beträgt 5 - 1000 ppm, vorzugsweise 10 - 500 ppm, besonders bevorzugt 20 - 250 ppm, bezogen auf die wässrige Phase. Die organische Phase kann als Tröpfchen durch Rühren bzw. durch Verdüsung (Jetting) in die wässrige Phase dispergiert werden. Unter organischer Phase wird das Monomerengemisch mit dem/den Initiator(en) verstanden.

Bei der klassischen Dispersionspolymerisation werden die organischen Tröpfchen durch Verrühren erzeugt. Im 4 Liter Maßstab werden typischerweise Rührerdrehzahlen von 250 bis 400 UpM verwendet. Werden die Tröpfchen durch Verdüsung erzeugt, empfiehlt es sich, zur Erhaltung des einheitlichen Tröpfchendurchmessers die organischen Tröpfchen zu verkapseln. Verfahren zur Mikroverkapselung von verdüsten organischen Tröpfchen werden beispielsweise in EP-A 0 046 535 beschrieben, deren Inhalt in Bezug auf die Mikroverkapselung von der vorliegenden Anmeldung mit umfasst wird.

Die mittlere Teilchengröße der gegebenenfalls verkapselten Monomertröpfchen beträgt 10-1000 µm, vorzugsweise 100-1000 µm.

Das Verhältnis der organischen Phase zur wässrigen Phase beträgt in der Regel 1:20 bis 1:0,6, bevorzugt 1:10 bis 1:1, besonders bevorzugt 1:5 bis 1:1,2.

Die organische Phase kann aber auch, gemäß EP-A 0 617 714 deren Lehre von der vorliegenden Anmeldung mit umfasst wird, im so genannten Seed-Feed-Verfahren zu einer Suspension von Saatpolymerisaten, die die organische Phase aufnehmen, zugegeben werden. Die mittlere Teilchengröße der mit der organischen Phase gequollenen Saatpolymerisate beträgt 5-1200 µm, vorzugsweise 20 - 1000 µm. Das Verhältnis der Summe organische Phase und Saatpolymerisat zur wässrigen Phase beträgt in der Regel 1:20 bis 1:0,6, bevorzugt 1:10 bis 1:1, besonders bevorzugt 1:5 bis 1:1,2.

Die Polymerisation der Monomeren wird bei erhöhter Temperatur durchgeführt. Die Polymerisationstemperatur richtet sich nach der Zerfallstemperatur des Initiators und liegt typischerweise im Bereich von 50 bis 150°C, vorzugsweise 60 bis 130°C. Die Polymerisationsdauer liegt bei 30 Minuten bis 24 Stunden, bevorzugt 2 bis 15 Stunden.

Am Ende der Polymerisation werden die vernetzten Perlpolymerisate von der wässrigen Phase abgetrennt, bevorzugt auf einer Nutsche, und gegebenenfalls getrocknet.

Die gemäß Schritt a) hergestellten vernetzten Perlpolymerisate werden in Schritt b) sulfoniert. Erfindungsgemäß wird die Sulfonierung in Schritt b) bei einer Konzentration der Schwefelsäure von mindestens 75 Gew.-% durchgeführt. Bevorzugt erfolgt die Sulfonierung in der Art, dass während der Reaktion die Konzentration der Schwefelsäure zwischen 80 Gew.-% und 98 Gew.-% liegt. Üblicherweise werden um diese Konzentrationen während der Sulfonierung zu erzielen, Schwefelsäuren mit einer Konzentration zwischen 80 Gew.-% und 100 Gew.-% eingesetzt. Falls die Schwefelsäure z.B. in einer Konzentration von 80 Gew.-% eingesetzt werden würde, würde der übrige Rest Wasser in einer Konzentration von 20 Gew.-% darstellen. Üblicherweise können auch Schwefelsäuren mit geringeren Konzentrationen eingesetzt werden und die Konzentration dann durch Zugabe von Schwefeltrioxid weiter erhöht werden. In diesen

Fällen könnte auch Schwefelsäure einer Konzentration von 60 Gew.-% eingesetzt werden und dann Schwefeltrioxid zugegeben werden, so dass die Konzentration der Schwefelsäure, während der Sulfonierungsreaktion mindestens 75 Gew. % beträgt oder80 Gew.-% bis 100 Gew.-% beträgt. Bevorzugt wird kein zusätzliches Schwefeltrioxid der Schwefelsäure in Schritt b) zugesetzt.

Erfindungsgemäß wird Schwefelsäure in Schritt b) in einer Konzentration von 92 Gew.-% bis 99 Gew.-% eingesetzt. Besonders bevorzugt liegt die Konzentration während der Sulfonierungsreaktion in Schritt b) zwischen 89 Gew.-% und 96 Gew.-% bei Einsatz einer Schwefelsäure mit einer Ausgangskonzentration von 92 Gew.-% bis 99 Gew.-%.

Es ist vorteilhaft in Schritt b), die notwendige Säurekonzentration durch Mischen von Schwefelsäure einer höheren und einer niedrigeren Konzentration einzustellen, wobei als Schwefelsäure mit niedrigerer Konzentration zurückgewonnene Schwefelsäure aus früheren Sulfonierungsreaktionen eingesetzt werden kann. Das Mischen der Schwefelsäure kann im Sulfonierungsreaktor in Anwesenheit des zu sulfonierenden Perl-polymerisats erfolgen, so dass die auftretende Mischungswärme zu einer Temperaturerhöhung des Reaktionsgemisches führt.

In Schritt b) sollte die Schwefelsäure in einer Menge von 70 Gew.-% bis 95 Gew.-% und das Perlpolymerisat in einer Menge von 5 Gew.-% bis 30 Gew.-% eingesetzt werden, wobei die Summe der Gewichtsprozente der Schwefelsäure und des Perlpolymerisates bezogen auf die Menge der Reaktionsmischung > 98 Gew. % ist. Der Rest zu 100 Gew.% könnten z.B. weitere organische Lösungsmittel oder nicht polymerisierter Monomerenreste darstellen. Das Sulfonierungsmittel in Schritt b) wird in einer Menge 70 Gew.-% bis 95 Gew.-% in einer Konzentration von 92 Gew.-% bis 99 Gew.-% eingesetzt, wobei die Menge des Perlpolymerisates dann zwischen 5 Gew.-% und 30 Gew.-% beträgt und die Summe der Gewichtsprozente der Schwefelsäure und des Perlpolymerisates bezogen auf die Menge der Reaktionsmischung > 98 Gew.-% ergibt. Ganz besonders bevorzugt ist die Summe der Gewichtsprozente der Schwefelsäure und des Perlpolymerisates bezogen auf die Menge der Reaktionsmischung 100 Gew. %.

Schritt b) des erfindungsgemäßen Verfahrens wird bevorzugt in Abwesenheit eines Quellungsmittels, wie insbesondere 1,2-Dichlorethan, durchgeführt. Als Quellungsmittel gelten alle organischen aliphatischen oder aromatischen Lösungsmittel. Besonders bevorzugt sind Quellungsmittel im Sinne der Erfindung 1,2-Dichlorethan, Methylenchlorid und Dichlorbenzoyl. Bevorzugt in Abwesenheit eines Quellungsmittel bedeutet im Sinne der Erfindung, dass die Menge an Quellungsmitteln in der Reaktionsmischung höchstens zwischen 1 Gew. % bis < 4 Gew, %, ganz besonders bevorzugt < 1 Gew. % beträgt und noch weiter bevorzugt dass kein Quellungsmittel enthalten ist. Es hat sich gezeigt, dass die Perlpolymerisate während der Sulfonierung gemäß Schritt b) einen Durchmesser haben, der zwischen 5 bis 15 % geringer ist, als während der Sulfonierung in Anwesenheit eines Quellungsmittels.

Die Temperatur bei der Sulfonierung in Schritt b) liegt bevorzugt bei 90°C bis 140 °C.

Es kann vorteilhaft sein, in Schritt b) ein Temperaturprogramm anzuwenden, in dem die Sulfonierung in einem ersten Reaktionsschritt bei einer ersten Temperatur begonnen wird und in einem zweiten Reaktionsschritt bei einer höheren Temperatur fortgeführt wird.

Bevorzugt wird die Reaktionsmischung zunächst zwischen 10 min und 60 min bei 90 °C bis 110 °C gerührt und dann auf 120 °C bis 140 °C erhitzt und weitere 3 bis 7 Stunden bei gleichbleibender Temperatur erwärmt.

Bei der Sulfonierung in Schritt b) wird das Reaktionsgemisch gerührt. Dabei können verschiedene Rührertypen, wie Blatt-, Anker-, Gitter- oder Turbinenrührer eingesetzt werden.

Die Dauer der Sulfonierungsreaktion in Schritt b) beträgt im Allgemeinen mehrere Stunden, bevorzugt zwischen 1 und 24 h, besonders bevorzugt zwischen 2 und 16 h, ganz besonders bevorzugt zwischen 3 und 12 h.

Nach der Sulfonierung in Schritt b) kann das Reaktionsgemisch aus Sulfonierungsprodukt und Restsäure zunächst auf Raumtemperatur abgekühlt werden und dann mit Schwefelsäuren abnehmender Konzentrationen und dann mit Wasser verdünnt werden. Die sulfonierten, vernetzten Perlpolymerisate aus Schritt b) des erfindungsgemäßen Verfahrens stellen stark saure Kationenaustauscher dar, die gegebenenfalls weiter gereinigt werden können, bevor sie in Schritt c) eingesetzt werden. Die Reinigung kann mit entionisiertem Wasser bei Temperaturen von 70 - 180 °C, vorzugsweise von 70 - 130 °C, besonders bevorzugt 70 °C bis 100 °C durchgeführt werden. Bevorzugt werden die sulfonierten, vernetzten Perlpolymerisate aus Schritt b) zunächst gereinigt, bevor sie in Schritt c) weiter umgesetzt werden.

Als Thioalkohole können in Schritt c) sämtliche azyklischen und zyklischen, verzweigten oder unverzweigten, gesättigten oder ungesättigten, aliphatischen oder aromatischen Kohlenwasserstoffverbindungen mit mindestens einer oder mehrere Thiolgruppen eingesetzt werden. Beispielsweise und vorzugsweise können als Thioalkohole, Aminoalkanthiole, wie z.B. Aminoethanthiol, Aminopropanthiol, Aminobutanthiol oder Aminopentanthiol oder Alkyl-Aminoalkanthiole wie z.B. Propylaminopropanthiol, Propylaminobutanthiol oder Propylaminoethanthiol oder Dialkyl-Aminoalkanthiole, wie z.B. Dimethylaminoethanthiol oder Mercaptoalkylamide, wie z.B. N-(2-Mercaptoethyl)propionamid oder Aminoalkanphosphonate, N-Alkyl-N-Mercaptoalkyl-mercaptoalkylaniline, wie z.B. N-(2-Mercaptoethyl)-4-(2-mercaptoethyl)-anilin, N-(2-Mercaptoethyl-N-methyl-4-(2-Mercapto-ethyl)-anilin, N-Ethyl-N-(2-mercaptoethyl)-4-(2-Mercaptoethyl)-anilin, N-(2-Mercaptopropyl)-4-(2-mercaptoethyl)-anilin, N-(2- Mercaptopropyl)-N-methyl-4-(2-mercaptoethyl)-anilin, N-Ethyl-N-(2-mercaptopropyl)-4-(2- mercaptoethyl)-anilin, N-(2-Mercaptoethyl)-4-(2-mercaptopropyl)-anilin, N-(2-Mercaptoethyl)-N-methyl-4-(2-mercaptopropyl)-anilin, N - Ethyl-N -(2-mercaptoethyl)-4 -(2-mercaptopropyl)-anilin, N -(2-Mercaptopropyl)-4-(2-Mercaptopropyl)-anilin, N-(2-Mercaptopropyl)-N-methyl-4-(2-mercaptopropyl)-anilin, N-Ethyl-N-(2-Mercaptopropyl)-4-(2-mercaptopropyl)-anilin oder Mercaptoalkylphenylpyridine, wie z.B. 2-(4-Mercapto-methylphenyl)-pyridin, 3-(4-Mercapto-methylphenyl) pyridin, 2-(3-Mercapto-methyl -phenyl) pyridin, 3-(3- Mercapto-methyl-phenyl)-pyridin, 4-(3-Mercapto-methyl-phenyl)pyridin, 2-(2-Mercapto-methyl-phenyl) pyridin, 3-(2 -Mercapto-methyl-phenyl)pyridin, 4-(2-Mercapto-Methyl-phenyl)pyridin, 2-(4-(2-Mercapto-ethyl) phenyl)pyridin, 3-(4-(2-Mercapto-ethyl)-phenyl)-pyridin, 4-(4-(2-Mercapto-ethyl)phenyl) pyridin, 2-(3-(2-Mercapto-ethyl)phenyl)pyridin, 3-(3-(2-Mercapto-5-ethyl) phenyl) pyridin, 4-(3-(2- Mercapto-ethyl) phenyl) pyridin, 2-(2-(2-Mercapto-ethyl) phenyl) pyridin, 3-(2-(2-Mercapto-ethyl)-phenyl)-pyridin, 4-(2-(2-Mercapto-ethyl)-phenyl)-pyridin oder Pyridinalkanthiole wie z.B. 4-Pyridinmethanthiol, 3-Pyridinmethanthiol, 2-(4- Pyridyl)ethanthiol, 2-(2-Pyridyl)ethanthiol, 2-(3-Pyridyl)ethanthiol, 3-(4-Pyridyl)propanthiol, 3-(3-Pyridyl)-propanthiol, 3-(4-Pyridyl)propanthiol, 4-(4-Pyridyl)butanthiol, 4-(3-Pyridyl)butanthiol, 4-(2-Pyridyl)butanthiol oder Mercaptoalkylbenzylamine, Imidazolalkylthiole, Phthalimidinalkylthiol, wie z.B. s-Acetyl-n-(2'-mercaptoethyl)phthalimidin oder Aminothiophenole oder beliebigen Mischung dieser Verbindungen.

Als Thioester werden beispielweise und vorzugsweise Pyridinalkylthioester, wie z.B. 2-(2'-thioacetatethyl)pyridin, 4-(2'-thioacetatethyl)pyridin oder Imidazolalkylthioester, wie z.B. 2-Mercaptoethylbenzimidazol oder Phthalimidinalkylthioester, wie z.B. N,S-Diacetyl-2-mercaptoethylbenzimidazol oder Gemische dieser Verbindungen eingesetzt.

Als Thioether werden beispielweise und vorzugsweise Pyridinalkylsulfide wie z.B. 2-(2'-tert-butylthioethyl)pyridin, 4-(2'-tert-butylthioethyl)pyridin, Imidazoalkylsulfid, Polyschwefelthioalkylverbindungen wie z.B. 2-(6'-tert-butylthiohexylthio)pyridin, 2-(4' -tert-butylthiobutylthio)pyridin, 2-(5'-tert-butylthiopentylthio)pyridin, 2-(3'-tert-butylthiopropyl-thio) pyridin, 4-(3'-tert-butylthiopropylthio)pyridin, Polyschwefelthiopyridin wie z.B. 2-(3'-tert-butylthiopropylthioethyl)pyridin, 4-(6'-tert-butylthiohexylthioethyl)pyridin, 4-(4'-tert-5-butylthiobutylthioethyl)pyridin, 4-(5'-tert-butylthiopentylthioethyl)pyridin, 4-(3'-tert-butyl-thiopropyl-thioethyl)pyridine, Polyschwefelthiobenzothiazol, Polyschwefelthioimidazol, oder Thiazolidin oder dessen Derivate, wie z.B. 3-Methylthiazolidin, 2-Methyl-2-ethylthiazolidin, 2-Methyl-2-dodecylthiazolidin, 2-Methyl-2-carbethoxymethylthiazolidin, 2,2,4,5-tetramethylthiazolidin, 2,2,3-Trimethylthiazolidin, 2,2-Dimethyl-3-octylthiazolidin, 2-Methyl-2-Ethyl-3-aminoethylthiazolidin, 2-Cyclohexylthiazolidin, 2,2'-Dimethylthiazolidin und beliebigen Mischungen dieser Verbindungen eingesetzt.

Besonders bevorzugt werden als schwefelhaltige Verbindungen in Schritt c) Aminoalkylthiole, wie insbesondere Aminoethanthiol, Aminopropanthiol, Aminobutanthiol oder Aminopentanthiol oder Thiazolidin oder dessen Derivate, wie insbesondere, 3-Methylthiazolidin, 2-Methyl-2-ethylthiazolidin, 2-Methyl-2-dodecylthiazolidin, 2-Methyl-2-carbethoxymethylthiazolidin, 2,2,4,5-tetramethylthiazolidin, 2,2,3-trimethylthiazolidin, 2,2-Dimethyl-3-octylthiazolidin, 2-Methyl-2-Ethyl-3-aminoethylthiazolidin, 2-Cyclohexylthiazolidin oder 2,2'-Dimethylthiazolidin oder Pyridinalkanthiole wie insbesondere 4-Pyridinmethanthiol, 3-Pyridinmethanthiol, 2-(4-Pyridyl)ethanthiol, 2-(2-Pyridyl)ethanthiol, 2-(3-Pyridyl)ethanthiol, 3-(4-Pyridyl)propanthiol, 3-(3-Pyridyl)propanthiol, 3-(4-Pyridyl)-propanthiol, 4-(4-Pyridyl)butanthiol, 4-(3-Pyridyl)butanthiol oder 4-(2-Pyridyl)butanthiol oder Gemische dieser Verbindungen eingesetzt.

Ganz besonders bevorzugt werden in Schritt c) als schwefelhaltige Verbindungen Dimethylthiazolidine, wie insbesondere 2,2'-Dimethylthiazolidin, Aminoethanthiol und 4-Pyridinethanthiol oder dessen Isomere oder Gemische dieser Verbindungen eingesetzt.

Die in Schritt c) eingesetzten schwefelhaltigen Verbindungen können ebenfalls in ihrer Salzform, also z.B. als Säure-Base Addukte in Gegenwart von Salzsäure oder Schwefelsäure oder anderen anorganischer oder organischer Säuren, eingesetzt werden.

Die Gesamtmenge an stark sauren Gruppen, die in dem sulfonierten, vernetzten Perlpolymerisat aus Schritt b) vorliegt, wird bevorzugt nur teilweise mit den schwefelhaltigen Verbindungen beladen. Bezogen auf die Gesamtmenge an sauren Gruppen in der Masse an sulfonierten, vernetzten Perlpolymerisat aus Schritt b) in mol gleich 100 % gesetzt, werden zwischen 5 und 45 mol %, bevorzugt zwischen 15 und 30 mol % an schwefelhaltigen Verbindungen eingesetzt.

Schritt c) kann sowohl im Säulenverfahren als auch im Batchverfahren durchgeführt werden. Im Batchverfahren, das bevorzugt angewendet wird, erfolgt die Beladung in Wasser oder in organischen Medien oder in Mischungen davon. Schritt c) des erfindungsgemäßen Verfahrens wird beispielsweise so durchgeführt, dass zunächst die sulfonierten, vernetzten Perlpolymerisate aus Schritt b) in Wasser oder anderen organischen Medien oder aber auch direkt aus Schritt b) kommend, ohne weiteren Zusatz von Flüssigkeiten, vorgelegt werden und dann die Mischung inertisiert wird. Beispielsweise werden die sulfonierten, vernetzten Perlpolymerisate aus Schritt b) durch Zugabe von Stickstoff oder anderen Inertgasen, wie z.B. Argon inertisiert. Beispielsweise erfolgt dann die Zugabe der schwefelhaltigen Verbindung in Schritt c), beispielsweise dosiert, unter Rühren. Es kann aber ebenfalls die Gesamtmenge der schwefelhaltigen Verbindung in Schritt c) auf einmal zu dem sulfonierten, vernetzten Perlpolymerisat aus Schritt b) zugegeben werden. Bevorzugt erfolgt die Zugabe dosiert. Danach kann die Mischung beispielsweise zwischen 30 min und 10 Stunden gerührt werden. Bevorzugt wird die Mischung zwischen 2 h und 6 h gerührt. Beispielsweise kann dann die Aufarbeitung der Reaktionsmischung in Schritt c) durch Zugabe von inertisiertem Wasser erfolgen. Beispielsweise kann zudem weiteres Inertgas dieser Mischung zugesetzt werden. Vorzugsweise wird die Mischung durch Zugabe von Stickstoff inertisiert, es können aber auch andere Inertgase eingesetzt werden. Vorzugsweise wird die Mischung zwischen 1 min und 10 min mit dem Inertgas in Schritt c) inertisiert. Die Mischung kann aber ebenfalls kürzer oder länger mit dem Inertgas inertisiert werden.

Vorzugsweise wird Schritt c) so durchgeführt, dass zunächst die sulfonierten, vernetzten Perlpolymerisate vorgelegt werden und dann durch Zugabe eines Inertgases inertisiert werden. Danach wird vorzugsweise die schwefelhaltige Verbindung unter Rühren dosiert zugegeben. Danach wird vorzugsweise für einen Zeitraum von 2 h bis 6 h weiter gerührt. Dann erfolgt vorzugsweise die Zugabe von inertisiertem Wasser. Danach wird vorzugsweise weiteres Inertgas, vorzugsweise Stickstoff, zur Inertisierung der Mischung in Schritt c) eingesetzt.

Schritt c) wird bevorzugt bei Temperaturen zwischen 5 °C und 80 °C, noch weiter bevorzugt bei Temperaturen zwischen 10 und 30 °C durchgeführt.

Die Lagerung des im erfindungsgemäßen Verfahrens hergestellten Katalysators erfolgt vorzugsweise unter Schutzgas.

Da der gemäß des erfindungsgemäßen Verfahrens hergestellte Katalysator eine besonders geringe TOC Menge innerhalb von 20 h an ein wässriges Medium abgibt, ist der Katalysator mit einer TOC (Gesamt organischer Kohlenstoff) Abgabemenge kleiner oder gleich 3 ppm, bevorzugt zwischen 1 ppm und 3 ppm, ebenfalls von der Erfindung mit umfasst. Ein wässriges Medium im Sinne der Erfindung stellt bevorzugt vollentsalztes Wasser dar. Der TOC Gehalt wird erfindungsgemäß wie folgt bestimmt:
Der Katalysator wird viermal mit Wasser gewaschen und im direkten Anschluss an die Behandlung in eine beheizbare Glasfiltersäule gefüllt. Die Temperatur der Filtersäule wird auf 70°C eingestellt. Mittels einer Schlauchpumpe wird nun in einem Zeitraum von 20 h abgekochtes VE-Wasser mit einer Geschwindigkeit von 0,2 BV/h über den Ionenaustauscher gepumpt.

Das Eluat wird aufgefangen und portionsweise in Glasflaschen gesammelt. Im vierten aufgefangenen Eluatbettvolumen , wird der TOC Gehalt analysiert.

Der mittlere Perldurchmesser der erfindungsgemäß hergestellten Katalysatoren liegt zwischen 30 µm und 2000 µm, bevorzugt zwischen 500 und 1000 µm, besonders bevorzugt zwischen 500 und 800 µm. Die erfindungsgemäß hergestellten Katalysatoren können in heterodisperser oder monodisperser Form hergestellt werden. Bevorzugt werden monodisperse Katalysatoren hergestellt. Die erfindungsgemäß hergestellten Katalysatoren weisen gelartige Eigenschaften auf und werden daher auch als gelförmige Katalysatoren bezeichnet.

Als monodispers werden in der vorliegenden Anmeldung solche Stoffe bezeichnet, bei dem mindestens 90 Volumen- oder Massen-% der Teilchen einen Durchmesser besitzen, der in dem Intervall mit der Breite von + 10 % des häufigsten Durchmessers um den häufigsten Durchmesser herum liegt.

Zum Beispiel bei einem Stoff mit häufigstem Durchmesser von 0,5 mm liegen mindestens 90-Volumen- oder Massen-% in einem Größenintervall zwischen 0,45 mm und 0,55 mm, bei einem Stoff mit häufigstem Durchmesser von 0,7 mm liegen mindestens 90 Volumen- oder Massen-% in einem Größenintervall zwischen 0,77 mm und 0,63 mm.

Die Katalysatoren können bei Kondensations-, Additions- und Veresterungsreaktionen, wie aus der DE 10027908 A1 bekannt ist, deren Inhalt bezüglich dieser Reaktionen von der vorliegenden Patentanmeldung mit umfasst wird, eingesetzt werden.

Bevorzugt werden die gelförmigen Katalysatoren bei Kondensationsreaktionen zur Synthese von Bisphenolen ausgehend von Phenolen, o-, m-, p-Kresolen oder alpha- und beta-Naphtholen und Ketonen, wie z.B. und vorzugsweise Aceton, Acetophenon, Butanon, Hexafluoraceton oder Cyclohexanon, besonders bevorzugt zur Synthese von Bisphenol A (2,2-Bis( 4-hydroxyphenyl)-propan (BPA)) aus Phenol und Aceton, eingesetzt. Von der Erfindung ist daher ebenfalls die Verwendung der erfindungsgemäß hergestellten Katalysatoren zur Herstellung von Bisphenolen aus Phenolen und Ketonen, bevorzugt zur Herstellung von Bisphenol A aus Phenol und Aceton, umfasst.

Durch das erfindungsgemäße Verfahren können erstmals Bisphenol Katalysatoren, insbesondere Bisphenol A Katalysatoren, ohne Einsatz umweltschädlicher Quellungsmittel hergestellt werden. Zudem hat sich gezeigt, dass diese Katalysatoren eine besonders hohe Totalkapazität aufweisen. Weiterhin ermöglicht das erfindungsgemäße Verfahren die Herstellung von Katalysatoren, die eine verringerte TOC Abgabe aufweisen und daher auch aus diesem Grunde ökotoxikologisch bevorzugt sind.

### Beispiele

### Messmethoden

### Bestimmung der vom Katalysator in das wässrige Eluat abgegebenen Säuremenge

In eine Glassäule mit Bodenfritte werden 50 ml Katalysator mit voll entsalztem Wasser eingefüllt. Das Wasser wird bis auf Harzbetthöhe abgelassen. Dann werden weitere 10 ml Wasser dosiert. Das Harz lässt man 24 Stunden lang ruhen. Hiernach wird das Harz mit voll entsalztem Wasser eluiert - Fließgeschwindigkeit 80 ml pro Stunde. Das Eluat wird in 20 ml Portionen aufgefangen und mit 0,01 molarer Natronlauge titriert.

### Bestimmung des TOC Gehaltes

### Vorbehandlung

100 mL Harz werden in der H+-Form unter VE-Wasser eingerüttelt. Dann wird das Harz in ein 600 mL Becherglas überführt und das Wasser abgesaugt. 400 mL VE-Wasser werden ins Becherglas gegeben und wieder abgesaugt. Dieser Vorgang wird insgesamt 4-mal durchgeführt.

### Prüfung

Der vorbehandelte Ionenaustauscher wird im direkten Anschluss an die Vorbehandlung in die beheizbare Glasfiltersäule gefüllt. Die Temperatur der Filtersäule wird auf 70°C eingestellt. Mittels einer Schlauchpumpe wird nun in einem Zeitraum von 20 h abgekochtes VE-Wasser mit einer Geschwindigkeit von 0,2 BV/h über den Ionenaustauscher gepumpt.

Das Eluat wird aufgefangen und portionsweise in Glasflaschen gesammelt. Im vierten aufgefangenen Eluatbettvolumen , wird der TOC Gehalt analysiert.

Die Angabe erfolgt in mg TOC pro Liter Flüssigkeit.

### Bestimmung der Höhe der Totalkapazität

In ein 200 ml Becherglas werden bei 25 °C 100 ml voll entsalztes Wasser dosiert.

Dazu werden 20 ml Harz in der Wasserstoffform dosiert. Anschließend werden 5 Gramm NaCl p.a. dosiert.

Die Suspension wird 5 Minuten gerührt. Anschließend wird mit 1 n Natronlauge im Titrator titriert.

Der Laborautomat rechnet über den Natronlaugeverbrauch die Höhe der Totalkapazität in mol stark saure Gruppen pro Liter Harz aus.

### Beispiel 1

### Herstellung eines gelförmigen, monodispersen, vernetzten Perlpolymerisates

In einem 4 l Glasreaktor ausgerüstet mit Rührer, Kühler, Thermoelement, Stickstoffgaszufuhr werden 1160 ml entionisiertem Wasser vorgelegt. Dazu werden 3,59 g Borsäure und 0,99 g Natriumhydroxid dosiert und gelöst.

In diese Lösung werden 300 Gramm eines mikroverkapselten kugelförmigen Styrolpolymerisates mit einem Gehalt an copolymerisiertem Divinylbenzol von 1,0 Gew.% als Saat dispergiert. Die Mikrokapselwand besteht aus einem mit Formaldehyd gehärtetem Komplexkoazervat aus Gelatine und einem Acrylamid/Acrylsäure - Copolymerisat.

Dann wird innerhalb von 30 Minuten bei Raumtemperatur eine Mischung von 847 Gramm Styrol, 48,75 Gramm Divinylbenzol 80 gew. %-ig - handelsübliches Gemisch aus Divinylbenzol , Ethylstyrol und Ethylbenzol - und 4,5 Gramm Trigonox 21 S dosiert. Die Suspension rührt weitere 2 Stunden bei Raumtemperatur. Danach werden in 30 Minuten 100 Gramm einer 2 gew. % igen wässrigen Lösung von Walocel MT 400 dosiert. Die Suspension wird in 90 Minuten auf 63°C erhitzt und weitere 10 Stunden bei 63 °C gerührt.

Anschließend wird in 60 Minuten auf 95 °C erhitzt und weitere 2 Stunden bei 95 °C gerührt.

Nach dem Abkühlen wird die Suspension in einen 10 Liter Reaktor, in dem 4 Liter voll entsalztes Wasser vorgelegt sind, dosiert. Es wird 5 Minuten gerührt. Die Suspension wird auf eine Nutsche gegeben .Das erhaltene Perlpolymerisat wird bei 70 °C 4 Stunden lang getrocknet.

Ausbeute an Perlpolymerisat nach Trocknung : 1193 Gramm

### Beispiel 2

### Herstellung eines stark sauren Kationenaustauschers ohne Einsatz des Quellmittels 1,2-Dichlorethan während der Sulfonierung

### Apparatur:

3 Liter Doppelwandplanschliffreaktor; Thermostat HP 4; KPG Gitterrührer; Messtropftrichter ; Feststofftrichter ; Messdatenerfassung

Bei Raumtemperatur werden 845 Gramm 98 gew. %-ige Schwefelsäure vorgelegt. Die Säure wird auf 100 °C erhitzt. Innerhalb von 15 Minuten werden 100 Gramm monodisperses Perlpolymerisat hergestellt wie in Beispiel 1 dosiert. Es wird mit einer Rührgeschwindigkeit von 150 UpM gerührt. Dann wird in einer Stunde auf 135°C erhitzt und weitere 5 Stunden bei dieser Temperatur gerührt.

Nach dem Abkühlen auf Raumtemperatur wird die Reaktionsmasse mit 78 gew.-% iger Schwefelsäure aus dem Reaktor in eine Säule gespült.

Über die in der Säule sich befindende Reaktionsmasse wird Schwefelsäure abnehmender Konzentration beginnend mit 78 gew.-%iger Schwefelsäure filtriert. Zum Schluss wird Wasser filtriert.

Liegt der Kationenaustauscher wasserfeucht vor, so wird bei 70 °C ein Bettvolumen voll entsalztes Wasser in einer Stunde filtriert. Danach ruht der Kationenaustauscher 1 Stunde bei 70 °C. Danach werden in 2 Stunden 2 Bettvolumen voll entsalztes Wasser bei 70°C über den Kationenaustauscher filtriert.

Dann wird der Kationenaustauscher auf Raumtemperatur abgekühlt.
Volumenausbeute : 675 ml
Trockengewicht : 0,2646 Gramm pro ml Kationenaustauscher
Totalkapazität Wasserstoffform : 1,35 mol/l
Totalkapazität Natriumform : 1,46 mol/l

Insgesamt werden pro Liter Harz 0,4 mmol Säure eluiert.

### Beispiel 3

### Herstellung eines stark sauren Kationenaustauschers mit Einsatz des Quellmittels 1,2-Dichlorethan während der Sulfonierung

### Apparatur:

3 Liter Doppelwandplanschliffreaktor; Thermostat HP 4; KPG Gitterrührer; Messtropftrichter; Feststofftrichter; Messdatenerfassung.

Bei Raumtemperatur werden 623 Gramm 85 gew.-%ige Schwefelsäure vorgelegt. Innerhalb von 15 Minuten werden 100 Gramm monodisperses Perlpolymerisat hergestellt wie in Beispiel 1 dosiert. Es wird mit einer Rührgeschwindigkeit von 150 UpM gerührt. Innerhalb von 5 Minuten werden 79 ml 1,2-Dichlorethan dosiert. Es wird 30 Minuten bei 25 °C dosiert. Dann werden 230 Gramm 65-gew.-%iges Oleum bei Raumtemperatur in 30 Minuten dosiert. Hierbei steigt die Temperatur auf 55 °C an. Dann wird in einer Stunde auf 115°C erhitzt und weitere 3 Stunden bei 115°C gerührt. Hierbei destilliert 1,2-Dichlorethan ab. Es wird Druckluft durchgeleitet, das verbliebenes 1,2-Dichlorethan austreibt. Dann wird in 30 Minuten auf 135°C erhitzt und weitere 5 Stunden bei dieser Temperatur gerührt.

Nach dem Abkühlen auf Raumtemperatur wird die Reaktionsmasse mit 78 gew.-%iger Schwefelsäure aus dem Reaktor in eine Säule gespült.

Über die in der Säule sich befindende Reaktionsmasse wird Schwefelsäure abnehmender Konzentration beginnend mit 78 gew.-%iger Schwefelsäure filtriert. Zum Schluss wird Wasser filtriert.

Liegt der Kationenaustauscher wasserfeucht vor, so wird bei 70 °C ein Bettvolumen voll entsalztes Wasser in einer Stunde filtriert. Danach ruht der Kationenaustauscher 1 Stunde bei 70 °C. Danach werden in 2 Stunden 2 Bettvolumen voll entsalztes Wasser bei 70°C über den Kationenaustauscher filtriert.

Dann wird der Kationenaustauscher auf Raumtemperatur abgekühlt.
Volumenausbeute : 710 ml
Trockengewicht : 0,2511 Gramm pro ml Kationenaustauscher
Totalkapazität Wasserstoffform : 1,27 mol/l
Totalkapazität Natriumform : 1,37 mol/l

### Beispiel 4

### Herstellung eines monodispersen Katalysators durch Beladung eines stark sauren Kationenaustauschers mit 2,2'-Dimethylthiazolidin

Bezogen auf die in der eingesetzten Harzmenge vorhandene gesamte Säuremenge in mol werden 20 mol % 2,2'-Dimethylthiazolidin eingesetzt.

### Apparatur:

3 Liter Doppelwandplanschliffreaktor; Thermostat HP 4; KPG Gitterrührer; Messtropftrichter; Feststofftrichter; Messdatenerfassung, Gaseinleitungsrohr.

Bei Raumtemperatur werden 600 ml voll entsalztes Wasser vorgelegt.

Dazu werden 1000 ml Kationenaustauscher hergestellt wie in Beispiel 2 unter rühren dosiert. Hiernach wird 30 Minuten lang Stickstoff durch die Reaktionsmasse geleitet. Dann werden innerhalb von 30 Minuten bei Raumtemperatur 29,5 Gramm 2,2'-Dimethylthiazolidin dosiert. Es wird 4 Stunden bei Raumtemperatur nachgerührt.

Die Reaktionsbrühe wird abgezogen. 600 ml mit Stickstoff inertisiertes Wasser werden dosiert. Es wird 5 Minuten gerührt, wobei Stickstoff durch die Reaktionsmasse geleitet wird.

Der Katalysator wird in eine mit Stickstoff geflutete Glasflasche abgelassen und trocken gesaugt.10 Minuten lang wird Stickstoff durch die Reaktionsmasse geleitet.
Trockengewicht : 26,82 Gramm pro 100 ml Katalysator feucht
Totalkapazität Originalform :1,01 mol/l
Totalkapazität Natriumform :1,07 mol/l

### Ergebnis

**Tabelle 1**

| **Eluatnummer** | **Säuremenge in mmol pro Liter Harz** |
|---|---|
| Erstes Eluat | 0,08 |
| Zweites Eluat | 0 |

### Beispiel 5

### Herstellung eines monodispersen Katalysators durch Beladung eines stark sauren Kationenaustauschers mit 2,2'- Dimethylthiazolidin

Bezogen auf die in der eingesetzten Harzmenge vorhandene gesamte Säuremenge in mol werden 20 mol % 2,2'-Dimethylthiazolidin eingesetzt.

### Apparatur:

3 Liter Doppelwandplanschliffreaktor; Thermostat HP 4; KPG Gitterrührer; Messtropftrichter; Feststofftrichter; Messdatenerfassung, Gaseinleitungsrohr.

Bei Raumtemperatur werden 600 ml voll entsalztes Wasser vorgelegt.

Dazu werden 1000 ml Kationenaustauscher hergestellt wie in Beispiel 3 unter rühren dosiert. Hiernach wird 30 Minuten lang Stickstoff durch die Reaktionsmasse geleitet. Dann werden innerhalb von 30 Minuten bei Raumtemperatur 27,5 Gramm 2,2'-Dimethylthiazolidin dosiert. Es wird 4 Stunden bei Raumtemperatur nachgerührt.

Die Reaktionsbrühe wird abgezogen. 600 ml mit Stickstoff inertisiertes Wasser werden dosiert. Es wird 5 Minuten gerührt, wobei Stickstoff durch die Reaktionsmasse geleitet wird.

Der Katalysator wird in eine mit Stickstoff geflutete Glasflasche abgelassen und trocken gesaugt.10 Minuten lang wird Stickstoff durch die Reaktionsmasse geleitet.
Trockengewicht : 23,02 Gramm pro 100 ml Katalysator feucht
Totalkapazität Originalform :0,96 mol/l
Totalkapazität Natriumform :1,04 mol/l

### Ergebnis

**Tabelle 2**

| **Eluatnummer** | **Säuremenge in mmol pro Liter Harz** |
|---|---|
| Erstes Eluat | 0,08 |
| Zweites Eluat | 0,02 |

Insgesamt werden pro Liter Harz 0,1 mmol Säure eluiert.

### Monodisperse Kationenaustauscher nicht mit 2,2'-Dimethylthiazolidin beladen

**Tabelle 3**

| | Sulfonierung | Totalkapazität | Wagnertest | | |
|---|---|---|---|---|---|
| Beispiel | | mol/l | Leitfähigkeit 4BV | Leitfähigkeit 2BV | TOC (Elution4BV) |
| | | | | | |
| 2 | ohne 1,2-Dichlorethan (DCE) | 1,35 | 18,74 µS/cm | 26,03 µS/cm | 3,36 ppm |
| 3 | mit 1,2-Dichlorethan (DCE) | 1,27 | 36,94 µS/cm | 70,35 µS/cm | 7,95 ppm |

### Monodisperse Kationenaustauscher mit 2,2'-Dimethylthiazolidin beladen

**Tabelle 4**

| Beispiel | TOC (Elution 4BV ) | Totalkapazität | Totalkapazität | Eluierte Säuremenge in mmol pro Liter Katalysator |
|---|---|---|---|---|
| | | teilweise | teilweise | |
| | | H - Form in mol/l | Na - Form in mol/l | |
| | | | | |
| 4 ohne DCE | 2,87 ppm | 1,10 mol/l | 1,19 mol/l | 0,08 |
| 5 mit DCE | 4,33 ppm | 1,01 mol/l | 1,07 mol/l | 0,1 |

## Patentansprüche

1. Verfahren zur Herstellung eines Katalysators, **dadurch gekennzeichnet, dass**
a) Monomertröpfchen aus einem Gemisch enthaltend mindestens eine monoethylenisch ungesättigte, aromatische Verbindung, mindestens eine multiethylenisch ungesättigte Verbindung, mindestens einen Initiator zu einem vernetzten Perlpolymerisat umgesetzt werden und
b) das vernetzte Perlpolymerisat aus Schritt a) bei einer Temperatur von 50 °C bis 160 °C in Gegenwart von Schwefelsäure sulfoniert wird und die Konzentration der Schwefelsäure während der Reaktion mindestens 75 Gew. % beträgt und die Menge der eingesetzten Schwefelsäure 70 Gew. % bis 95 Gew.% und die Menge des eingesetzten Perlpolymerisates 5 Gew. % bis 30 Gew. % bezogen auf die Gesamtmenge an eingesetzter Schwefelsäure und Perlpolymerisat beträgt und die Schwefelsäure in einer Konzentration zwischen 92 % und 99 % eingesetzt wird, und die Summe der Gewichtsprozente aus Schwefelsäure und Perlpolymerisat bezogen auf die Menge der Reaktionsmischung > 98 Gew.-% ist und
c) die sulfonierten, vernetzten Perlpolymerisate aus Schritt b) mit mindestens einer schwefelhaltigen Verbindung aus der Gruppe Thioalkohole, Thioether und Thioester oder Gemische dieser Verbindungen umgesetzt werden.

2. Verfahren gemäß Anspruch 1, **dadurch gekennzeichnet, dass** in Verfahrenschritt a) als monoethylenisch ungesättigte, aromatische Verbindung Styrol, α-Methylstyrol, Vinyltoluol, Ethylstyrol, t-Butylstyrol, Chlorstyrol, Bromstyrol, Chlormethylstyrol oder Vinylnaphthalin oder Mischungen dieser Verbindungen eingesetzt werden.

3. Verfahren gemäß Anspruch 1 oder 2, **dadurch gekennzeichnet, dass** in Verfahrenschritt a) als multiethylenisch ungesättigte, aromatische Verbindung Divinylbenzol, Divinyltoluol, Trivinylbenzol, Octadien oder Triallylcyanurat oder Mischungen dieser Verbindungen eingesetzt werden.

4. Verfahren gemäß mindestens einem der Ansprüche 1 bis 3, **dadurch gekennzeichnet, dass** als eine monoethylenisch ungesättigte, aromatische Verbindung Styrol und als multiethylenisch ungesättigte Verbindung Divinylbenzol eingesetzt werden.

5. Verfahren gemäß mindestens einem der Ansprüche 1 bis 4, **dadurch gekennzeichnet, dass** Verfahrenschritt a) in Abwesenheit von Verbindungen ausgewählt aus der Gruppe Toluol, Ethylbenzol, Xylol, Cyclohexan, Oktan, Isooktan, Decan, Dodekan, Isododekan, Methylisobutylketon, Ethylacetat, Butylacetat, Dibutyl¬phtalat, n-Butanol, 4-Methyl-2-pentanol und n-Octanol vorzugsweise in Abwesenheit von Porogenen durchgeführt wird.

6. Verfahren gemäß mindestens einem der Ansprüche 1 bis 5, **dadurch gekennzeichnet, dass** die Temperatur in Schritt b) während der Sulfonierung 90°C bis 140 °C beträgt.

7. Verfahren gemäß mindestens einem der Ansprüche 1 bis 6, **dadurch gekennzeichnet, dass** die Sulfonierung in Schritt b) für einen Zeitraum von 3 bis 12 h durchgeführt wird.

8. Verfahren gemäß mindestens einem der Ansprüche 1 bis 7, **dadurch gekennzeichnet, dass** als schwefelhaltige Verbindungen Thiazolidin oder dessen Derivate, Aminoalkylthiole oder derivatisierte Pyridinthiole in Schritt c) eingesetzt werden.

9. Verfahren gemäß mindestens einem der Ansprüche 1 bis 8, **dadurch gekennzeichnet, dass** als schwefelhaltige Verbindungen 2,2'-Dimethylthiazolidin, Aminoethanthiol oder 4-Pyridinthiol oder Gemische dieser Verbindungen in Schritt c) eingesetzt werden.

10. Verfahren gemäß mindestens einem der Ansprüche 1 bis 9, **dadurch gekennzeichnet, dass** Schritt c) bei einer Temperatur von 10 °C bis 30°C durchgeführt wird.

11. Katalysator hergestellt gemäß einem der Ansprüche 1 bis 10, **dadurch gekennzeichnet, dass** der Katalysator eine Menge kleiner oder gleich 3 ppm gesamt organischer Kohlenstoff innerhalb von 20 h an ein wässriges Medium abgibt.

12. Verwendung des Katalysators aus Anspruch 11 zur Herstellung von Bisphenolen, insbesondere von Bisphenol A aus Phenol und Aceton.

## Claims

1. Process for preparing a catalyst, **characterized in that**
a) monomer droplets of a mixture comprising at least one monoethylenically unsaturated aromatic compound, at least one multiethylenically unsaturated compound and at least one initiator are converted to a crosslinked bead polymer, and
b) the crosslinked bead polymer from step a) is sulphonated in the presence of sulphuric acid at a temperature of 50°C to 160°C and the concentration of the sulphuric acid during the reaction is at least 75% by weight and the amount of sulphuric acid used is 70% by weight to 95% by weight and the amount of the bead polymer used is 5% by weight to 30% by weight, based on the total amount of sulphuric acid and bead polymer used, and the sulphuric acid is used in a concentration between 92% and 99%, and the sum total of the percentages by weight of sulphuric acid and bead polymer based on the amount of the reaction mixture is > 98% by weight, and
c) the sulphonated crosslinked bead polymers from step b) are reacted with at least one sulphur compound from the group of thioalcohols, thioethers and thioesters or mixtures of these compounds.

2. Process according to Claim 1, **characterized in that** the monoethylenically unsaturated aromatic compound used in process step a) is styrene, α-methylstyrene, vinyltoluene, ethylstyrene, t-butylstyrene, chlorostyrene, bromostyrene, chloromethylstyrene or vinylnaphthalene or mixtures of these compounds.

3. Process according to Claim 1 or 2, **characterized in that** the multiethylenically unsaturated aromatic compound used in process step a) is divinylbenzene, divinyltoluene, trivinylbenzene, octadiene or triallyl cyanurate or mixtures of these compounds.

4. Process according to at least one of Claims 1 to 3, **characterized in that** a monoethylenically unsaturated aromatic compound used is styrene and the multiethylenically unsaturated compound used is divinylbenzene.

5. Process according to at least one of Claims 1 to 4, **characterized in that** process step a) is performed in the absence of compounds selected from the group of toluene, ethylbenzene, xylene, cyclohexane, octane, isooctane, decane, dodecane, isododecane, methyl isobutyl ketone, ethyl acetate, butyl acetate, dibutyl phthalate, n-butanol, 4-methyl-2-pentanol and n-octanol, preferably in the absence of porogens.

6. Process according to at least one of Claims 1 to 5, **characterized in that** the temperature in step b) during the sulphonation is 90°C to 140°C.

7. Process according to at least one of Claims 1 to 6, **characterized in that** the sulphonation in step b) is conducted for a period of 3 to 12 h.

8. Process according to at least one of Claims 1 to 7, **characterized in that** the sulphur compounds used in step c) are thiazolidine or derivatives thereof, aminoalkylthiols or derivatized pyridinethiols.

9. Process according to at least one of Claims 1 to 8, **characterized in that** the sulphur compounds used in step c) are 2,2'-dimethylthiazolidine, aminoethanol or 4-pyridinethiol or mixtures of these compounds.

10. Process according to at least one of Claims 1 to 9, **characterized in that** step c) is conducted at a temperature of 10°C to 30°C.

11. Catalyst prepared according to any of Claims 1 to 10, **characterized in that** the catalyst releases an amount of less than or equal to 3 ppm of total organic carbon to an aqueous medium within 20 h.

12. Use of the catalyst from Claim 11 for preparation of bisphenols, especially of bisphenol A from phenol and acetone.

## Revendications

1. Procédé de fabrication d'un catalyseur, **caractérisé en ce que**
a) des gouttes de monomères d'un mélange contenant au moins un composé aromatique monoéthyléniquement insaturé, au moins un composé polyéthyléniquement insaturé, au moins un initiateur sont mises en réaction pour former un polymère en perles réticulé, et
b) le polymère en perles réticulé de l'étape a) est sulfoné à une température de 50 °C à 160 °C en présence d'acide sulfurique, et la concentration de l'acide sulfurique pendant la réaction est d'au moins 75 % en poids, et la quantité d'acide sulfurique utilisé est de 70 % en poids à 95 % en poids, et la quantité de polymère en perles utilisé est de 5 % en poids à 30 % en poids, par rapport à la quantité totale d'acide sulfurique et de polymère en perles utilisés, et l'acide sulfurique est utilisé en une concentration comprise entre 92 % et 99 %, et la somme des pourcentages en poids d'acide sulfurique et de polymère en perles par rapport à la quantité de mélange réactionnel est > 98 % en poids, et
c) les polymères en perles réticulés sulfonés de l'étape b) sont mis en réaction avec au moins un composé contenant du soufre du groupe constitué par les thioalcools, les thioéthers et les thioesters ou les mélanges de ces composés.

2. Procédé selon la revendication 1, **caractérisé en ce qu'**à l'étape de procédé a), du styrène, de l'α-méthylstyrène, du vinyltoluène, de l'éthylstyrène, du t-butylstyrène, du chlorostyrène, du bromostyrène, du chlorométhylstyrène ou de la vinylnaphtaline ou des mélanges de ses composés sont utilisés en tant que composés aromatique monoéthyléniquement insaturé.

3. Procédé selon la revendication 1 ou 2, **caractérisé en ce qu'**à l'étape de procédé a), du divinylbenzène, du divinyltoluène, du trivinylbenzène, de l'octadiène ou du cyanurate de triallyle ou des mélanges de ces composés sont utilisés en tant que composé aromatique polyéthyléniquement insaturé.

4. Procédé selon au moins l'une quelconque des revendications 1 à 3, **caractérisé en ce que** du styrène est utilisé en tant que composé aromatique monoéthyléniquement insaturé et du divinylbenzène en tant que composé polyéthyléniquement insaturé.

5. Procédé selon au moins l'une quelconque des revendications 1 à 4, **caractérisé en ce que** l'étape de procédé a) est réalisée en l'absence de composés choisis dans le groupe constitué par le toluène, l'éthylbenzène, le xylène, le cyclohexane, l'octane, l'isooctane, le décane, le dodécane, l'isododécane, la méthylisobutylcétone, l'acétate d'éthyle, l'acétate de butyle, le phtalate de dibutyle, le n-butanol, le 4-méthyl-2-pentanol et le n-octanol, de préférence en l'absence d'agents porogènes.

6. Procédé selon au moins l'une quelconque des revendications 1 à 5, **caractérisé en ce que** la température à l'étape b) pendant la sulfonation est de 90 °C à 140 °C.

7. Procédé selon au moins l'une quelconque des revendications 1 à 6, **caractérisé en ce que** la sulfonation à l'étape b) est réalisée pendant une durée de 3 à 12 h.

8. Procédé selon au moins l'une quelconque des revendications 1 à 7, **caractérisé en ce que** de la thiazolidine ou ses dérivés, des aminoalkylthiols ou des pyridine-thiols dérivés sont utilisés en tant que composés contenant du soufre à l'étape c).

9. Procédé selon au moins l'une quelconque des revendications 1 à 8, **caractérisé en ce que** de la 2,2'-diméthylthiazolidine, de l'aminoéthane-thiol ou du 4-pyridine-thiol ou des mélanges de ces composés sont utilisés en tant que composés contenant du soufre à l'étape c).

10. Procédé selon au moins l'une quelconque des revendications 1 à 9, **caractérisé en ce que** l'étape c) est réalisée à une température de 10 °C à 30 °C.

11. Catalyseur fabriqué selon l'une quelconque des revendications 1 à 10, **caractérisé en ce que** le catalyseur libère une quantité inférieure ou égale à 3 ppm de carbone organique total en 20 h dans un milieu aqueux.

12. Utilisation du catalyseur selon la revendication 11 pour la fabrication de bisphénols, notamment de bisphénol A à partir de phénol et d'acétone.
